# EUROPEAN PATENT SPECIFICATION

(11) **EP 2 480 226 B1**
(45) Date of publication and mention of the grant of the patent: **19.08.2015**
(21) Application number: 10819052.1
(22) Date of filing: 20.09.2010
(51) Int. Cl.: A61K 31/198, A61P 31/04, A61P 1/04, A23L 1/30, A23L 1/305, A61K 9/08, A61K 9/10, A61K 9/14, A61K 9/20, A61K 9/28, A61K 47/26, A61K 47/38, A61K 9/00, A61K 36/8962

(54) **COMPOSITION COMPRISING S-ALLYL-L-CYSTEINE AS ACTIVE INGREDIENT FOR PREVENTING OR TREATING DRUG-INDUCED GASTRIC MUCOSAL LESIONS**
ZUSAMMENSETZUNG MIT S-ALLYL-L-CYSTEIN ALS WIRKSTOFF ZUR VERHINDERUNG ODER BEHANDLUNG MEDIKAMENTENINDUZIERTER SCHÄDIGUNGEN DER MAGENSCHLEIMHAUT
COMPOSITION COMPRENANT S-ALLYL-L-CYSTÉINE À TITRE DE PRINCIPE ACTIF POUR PRÉVENIR OU TRAITER DES LESIONS DE LA MUQUEUSE GASTRIQUE INDUITES PAR MEDICAMENT

(30) Priority: 23.09.2009 KR 20090090232
(43) Date of publication of application: 01.08.2012
(73) Proprietor: Pharmaking Co., Ltd, Eumseong-gun, Chungcheongbuk-Do 369-852 (KR)
(72) Inventor: KIM, Soon Bae, Seongnam Si Gyeonggi-Do 463-500 (KR); KIM, Gwang Soon, Seongnam Si Gyeonggi-Do 463-744 (KR); KIM, Wan Bae, Seoul 110-797 (KR); KWAK, Wie Jong, Seoul 137-764 (KR); BANG, Sung Hye, Seoul 120-130 (KR)
(74) Representative: Ilgart, Jean-Christophe
(86) International application number: PCT/KR2010/006506
(87) International publication number: WO 2011/037411

(56) References cited:
- EP-A1- 0 195 433
- EP-A1- 0 429 080
- DE-A1- 3 514 995
- SIVAM G P ET AL: "Helicobacter pylori--in vitro susceptibility to garlic (Allium sativum) extract", NUTRITION AND CANCER, LONDON, GB, vol. 27, no. 2, 1 January 1997 (1997-01-01), pages 118-121, XP009117385, ISSN: 0163-5581
- WEI-CHENG YOU ET AL: "Helicobacter pylori Prevalence and CagA Status Among Children in Two Counties of China with High and Low Risks of Gastric Cancer", ANNALS OF EPIDEMIOLOGY, vol. 11, no. 8, 1 November 2001 (2001-11-01), pages 543-546, XP055050639, ISSN: 1047-2797, DOI: 10.1016/S1047-2797(01)00227-7
- MITCHELL H. GAIL ET AL: "Factorial Trial of Three Interventions to Reduce the Progression of Precancerous Gastric Lesions in Shandong, China", CONTROLLED CLINICAL TRIALS, vol. 19, no. 4, 1 August 1998 (1998-08-01) , pages 352-369, XP055050642, ISSN: 0197-2456, DOI: 10.1016/S0197-2456(98)00016-6
- MASAKI LIMURO ET AL. CANCER LETTERS vol. 187, 2002, pages 61 - 68, XP027335547
- YUKIHIRO KODERA ET AL. J. AGRIC. FOOD CHEM. vol. 50, 2002, pages 622 - 632, XP008151416
- GAIL MITCHELL H ET AL: "A factorial trial including garlic supplements assesses effect in reducing precancerous gastric lesions", THE JOURNAL OF NUTRITION, AMERICAN SOCIETY FOR NUTRITION, US, vol. 136, no. 3, SUPPL, 1 March 2006 (2006-03-01), pages 813s-815s, XP009168244, ISSN: 0022-3166
- YUKIHIRO KODERA ET AL: "Physical, Chemical, and Biological Properties of S-Allylcysteine, an Amino Acid Derived from Garlic", JOURNAL OF AGRICULTURAL AND FOOD CHEMISTRY, AMERICAN CHEMICAL SOCIETY, US, vol. 50, no. 3, 30 January 2002 (2002-01-30), pages 622-632, XP008151416, ISSN: 0021-8561, DOI: 10.1021/JF0106648 [retrieved on 2001-12-29]
- Ana L. Colín-González ET AL: "The Antioxidant Mechanisms Underlying the Aged Garlic Extract- and S-Allylcysteine-Induced Protection", Oxidative Medicine and Cellular Longevity, vol. 237, no. 1, 1 January 2012 (2012-01-01), pages 265-16, XP055131326, ISSN: 1942-0900, DOI: 10.1186/1471-2326-2-3

## Description

### Technical Field

### Cross-reference to related patent application

This application claims the benefit of Korean Patent Application No. 10-2009-0090232, filed on September 23, 2009, in the Korean Intellectual Property Office.

### Field of the invention

The present invention relates to a composition having a gastric mucosa protective effect, a composition for preventing or treating gastrointestinal disorders, and a method of using the compositions.

### Background Art

Gastrointestinal disorders are caused by a variety of factors and are known to be caused by an imbalance between aggressive factors, such as *Helicobacter pylori,* gastric acid, pepsin, overwork, stress, and alcohol, and defensive factors, such as mucus secretion, tissue-regenerative capability, and anticoagulant activity. Gastritis caused by overwork, stress, *Helicobacter pylori* infection, or the like is a common symptom but can develop, if not treated, into chronic gastritis, gastric ulcer, and, rarely, gastric cancer. A gastric mucosal lesion caused by alcohol can be healed within several days by the removal of the stimulus factor but may develop into gastrointestinal bleeding, a gastric perforation, or the like (Taeyoung Oh, et al., J. Applied Pharmacology, Vol. 5, pp202-210, 1997). Various drugs such as cimetidine, ranitidin, famotidine, omeprazole, or bismuth have been used for treating gastrointestinal disorders. However, the relapse rate after discontinuation of these drugs is very high, and thus there is a need to develop a novel drug.

*Helicobacter pylori* is a gram-negative bacterium that colonizes human gastric mucosa or mucus. It is recognized that *Helicobacter pylori* infection is a significant contributory factor in the development of most gastrointestinal tract-associated disorders such as acutechronic gastritis, atrophic gastritis, gastric ulcer, gastric cancer, and duodenal ulcers (Crowe, Curr Opin Gastrenterol., 21(1), pp32-38, 2005). Furthermore, it has been reported that *Helicobacter pylori* infection is a risk factor for hepatic encephalopathy, arteriosclerosis, and hepatobiliary system-associated diseases in addition to digestive system-associated diseases (Karahalil, et al., Curr Drug Saf, 2, pp43-46, 2007; Scragg, et al., J. Epidemiol Community Health, 50(5), pp578-579, 1996). According to the US Centers for Disease Control and Prevention (CDC), *Helicobacter pylori* infection may cause chronic fatigue, urticaria, migraine, short stature, infertility, food allergy, etc., which are all symptoms of'strange Helicobacter syndrome'. In general, antibiotics are used to kill *Helicobacter pylori.* However, *Helicobacter pylori* reinfection is common after eradication thereof and a high-dose treatment is required for a long period of time to completely remove the *Helicobacter pylori.* Accordingly, the long-term use of high dose antibiotics may lead to side effects and increase in antibiotic-resistant bacteria.

Recently, diverse research is being conducted into materials that inhibit the growth of bacteria in food as a safe method to treat *Helicobacter pylori* infection-associated diseases. Lactobacillus-fermented milk using probiotics, egg-yolk-derived immunoglobulin (IgY) including a *Helicobacter pylori-*neutralizing antibody, and catechin contained in wine and green tea are considered as effective substances against infection by *Helicobacter pylori*(McMahon, et al., Aliment Pharmacol Ther 23(8), pp1215-1223, 2006; Sachdeva, et al., Eur J Gastroenterol Hepatol, 21(1), pp45-53, 2009; Shin, et al., J Med Microbiol., 53(Pt 1), pp31-34, 2004).

Meanwhile, garlic(Allium Sativum.L) that belongs to the Allium genus has antimicrobial, antifungal, anti-oxidant, and anti-cancer properties (Ankri, et al., Microbes Infect. 1(2), pp125-129, 1999), prevents thrombosis, inflammation, and oxidative stress of cells (Sener, et al., Mol Nutr Food Res., 51(11), pp1345-1352, 2007), and thus has drawn attention. Garlic contains various components including steroidal saponin such as eruboside-B having antifungal and anti-cancer properties (Matsuura H, et al., Chem Pharm Bull (Tokyo), 36: 3659-3663, 1988), glycoside fractions having a cholesterol-lowering effect (Slowing, et al., J Nutr., 131, pp994S-9S, 2001), nonsulfur compounds such as β-chlorogenine having a platelet aggregation inhibiting effect (Rahman K, et al., J. Nutr. 2006), and various organosulfur compounds. Examples of the organosulfur compounds contained in plants belonging to the Allium genus are fat-soluble organosulfur compounds such as S-allyl-L-cysteine sulfoxides (alliin), Diallydisulfide (DADS), and Diallyl sulfide (DAS) and water-soluble organosulfur compounds such as S-allyl-L-cysteine (SAC) and S-allylmercaptocysteine (SAMC).

It has been reported that SAC, which is an active ingredient of mature garlic, has an anti-oxidant activity that inhibits arteriosclerosis and anticancer activity in some cancer cell lines (Proceedings of the American Association for Cancer Research, 30, p181, 1989).

DE 35 14995 A1 relates to sulfhydrylic compounds for use in combating gastric mucosa damages induced by anti-inflammatory, analgesic and antipyretic drugs such as acetylsalicylic acid and indomethacin. L-cysteine, L-cystine, acetylcysteine, cysteamine, DL-methionine, dilaurylthiodipropionate, 2-hydroxy-4-methylthiobutyrate, 3,3'-thiodipropionic acid, sodium thiosulfate, biotine and mixtures of these compounds are disclosed as sulfhydrylic compounds.

EP 0 195 433 A1 relates to amide derivatives for use in treating ulcers in digestive tracts and inflammation, such ulcers being possibly induced by aspirin and ethanol. These amide derivatives correspond to a sulfur-containing amine combined with a C₅-C₁₈ unsaturated aliphatic acid by one or more amide bonds.

It has not been reported that SAC has therapeutic effects for preventing or treating gastric mucosal lesions induced by ethanol, aspirin or indomethacin.

### Disclosure

### Technical Problem

In general, antibiotics are used to kill *Helicobacter pylori*. However, *Helicobacter pylori* reinfection is common after eradication thereof and high-dose treatment is required for a long period of time to completely remove the *Helicobacter pylori*. Accordingly, side effects may occur and antibiotic-resistant bacteria may increase due to the use of antibiotics. Therefore, there is a need for an alternative drug other than antibiotics. The present invention provides a drug having a gastric mucosa protective effect, as defined in the claims.

The present invention also provides a safe composition for preventing, relieving, or treating gastrointestinal disorders, as defined in the claims.

### Technical Solution

According to an aspect of the present invention, there is provided a composition comprising S-allyl-L-cysteine (SAC), which is a water-soluble organosulfur compound contained in a plant belonging to the Allium genus, as an active ingredient and having a mucosa protective effect, as defined in the claims. According to another aspect of the present invention, there is provided a composition including SAC as an active ingredient for preventing, relieving, or treating gastrointestinal disorders, as defined in the claims.

### Advantageous Effects of Invention

The composition including SAC as an active ingredient according to the present invention prevents and treats gastric mucosal lesions caused by hydrochloric acid-ethanol, aspirin, or indomethacin, and thus may be efficiently used to prevent, relieve, or treat gastrointestinal disorders. The composition including SAC as an active ingredient according to the present invention may be used as a pharmaceutical composition or food composition.

### Brief Description of Drawings

The above and other features and advantages of the present invention will become more apparent by describing in detail exemplary embodiments thereof with reference to the attached drawings in which:

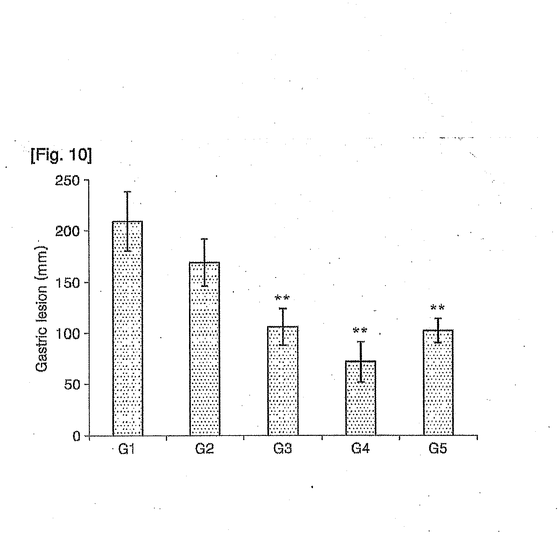

FIG. 10 shows an effect of SAC on lengths of gastric lesions of rats induced by hydrochloric acid-ethanol, wherein ** indicates a significant difference from a vehicle control group G1 by p<0.01, G1: vehicle control group (distilled water), G2: 100 mg/kg of SAC, G3: 200 mg/kg of SAC, G4: 400 mg/kg of SAC, and G5: positive control group (55.6 mg/kg of Stillen^{®} as an active ingredient);

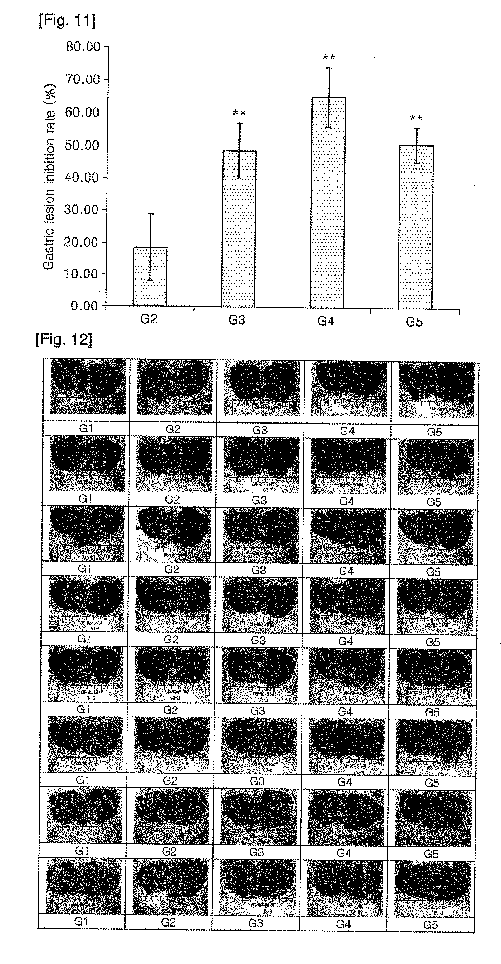

FIG. 11 shows gastric lesion inhibiting rates in rats in hydrochloric acid-ethanol-induced gastric lesion model, wherein ** indicates a significant difference from a vehicle control group G1 by p<0.01, G1: vehicle control group (distilled water), G2: 100 mg/kg of SAC, G3: 200 mg/kg of SAC, G4: 400 mg/kg of SAC, and G5: positive control group (55.6 mg/kg of Stillen^{®} as an active ingredient);
FIG. 12 shows photographs of stomachs of rats in hydrochloric acid-ethanol-induced gastric lesion model, wherein G1: vehicle control group (distilled water), G2: 100 mg/kg of SAC, G3: 200 mg/kg of SAC, G4: 400 mg/kg of SAC, and G5: positive control group (55.6 mg/kg of Stillen^{®} as an active ingredient);

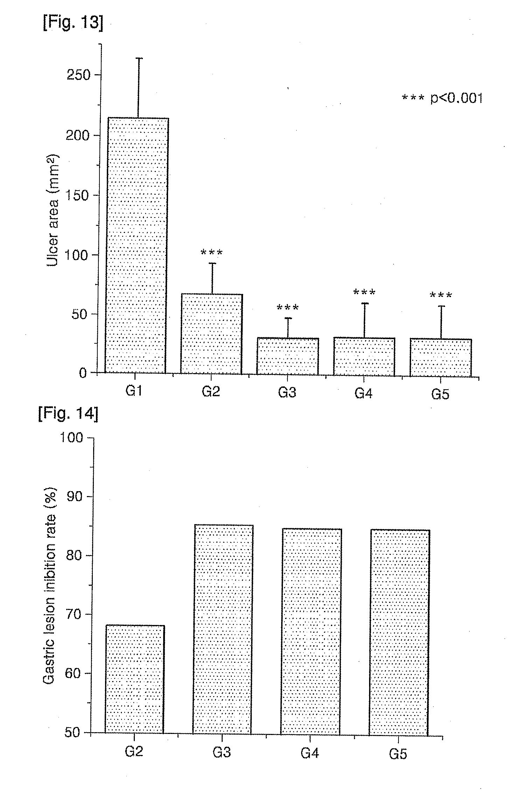

FIG. 13 shows an effect of SAC on an area of gastric lesions of rats induced by aspirin, wherein *** indicates a significant difference from a vehicle control group G1 by p<0.001, G1: vehicle control group (distilled water), G2: 100 mg/kg of SAC, G3: 200 mg/kg of SAC, G4: 400 mg/kg of SAC, and G5: positive control group (55.6 mg/kg of Stillen^{®} as an active ingredient);
FIG. 14 shows gastric lesion inhibiting rates in rats in an aspirin-induced gastric lesion model;

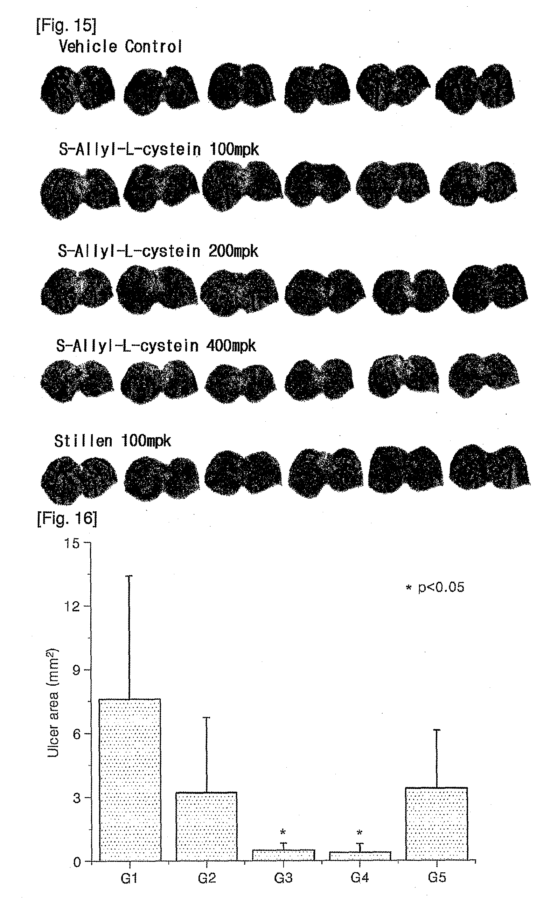

FIG. 15 shows photographs of stomachs of rats in an aspirin-induced gastric lesion model;
FIG. 16 shows an effect of SAC on lengths of gastric lesions of rats induced by indomethacin, wherein * indicates significant difference from a vehicle control group G1 by p<0.05, G1: vehicle control group (distilled water), G2: 100 mg/kg of SAC, G3: 200 mg/kg of SAC, G4: 400 mg/kg of SAC, and G5: positive control group (55.6 mg/kg of Stillen^{®} as an active ingredient);

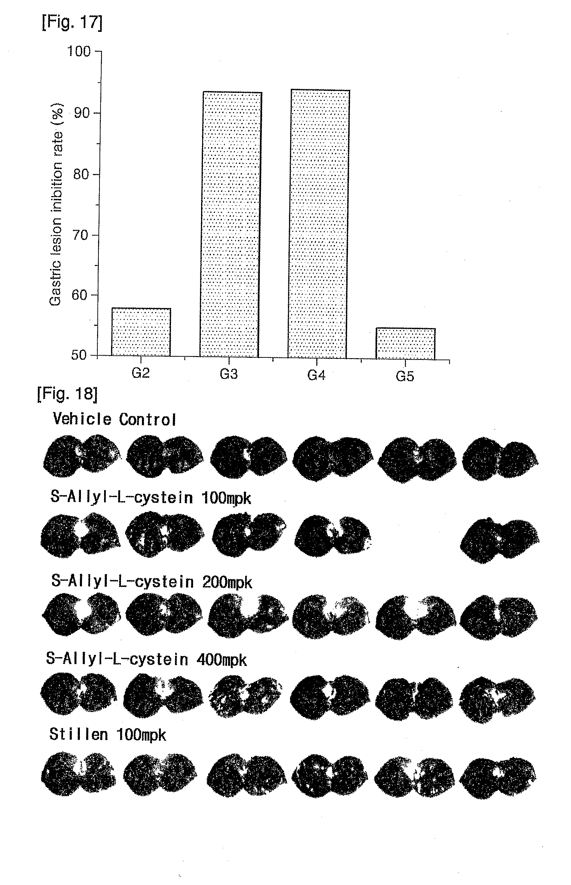

FIG. 17 shows gastric lesion inhibiting rates in rats in an indomethacin-induced gastric lesion model; and
FIG. 18 shows photographs of stomachs of rats in an indomethacin-induced gastric lesion model.

### Mode for the Invention

### DETAILED DESCRIPTION OF THE INVENTION

Hereinafter, the present invention will now be described more fully with reference to the accompanying drawings, in which exemplary embodiments of the invention is shown.

A composition including S-allyl-L-cysteine (SAC) has an excellent gastric mucosa protective effect, as defined in the claims.

The present inventors have found that SAC has a significant effect on inhibiting gastric mucosal lesions that were induced in the rats by a drug administration. In a gastric lesion induced in a rat by hydrochloric acid-ethanol, SAC exhibited a significant reduction in the lesion when compared to a control group to which only a vehicle was administered (FIGS. 10 and 12) and a gastric lesion inhibiting rate (%) up to about 66% (FIG. 11). SAC also exhibited a significant reduction in a gastric lesion induced by aspirin (FIGS. 13-15) or indomethacin (FIGS. 16-18) when compared to the negative control group. SAC has a gastric lesion inhibiting rate (%) up to about 85% in the gastric lesion induced by aspirin and a gastric lesion inhibiting rate (%) up to about 94% in the gastric lesion induced by indomethacin, which are similar or better gastric mucosa protective effects compared to Stillen^{®} used as a positive control group. The results indicate that SAC has diverse gastric mucosa protective effects on gastric lesion caused by various factors.

The present invention provides a pharmaceutical composition including SAC as an active ingredient with a pharmaceutically acceptable carrier and having a gastric mucosa protective effect, as defined in the claims.

The present invention provides a food composition including SAC as an active ingredient and having a gastric mucosa protective effect, as defined in the claims.

The present invention provides a pharmaceutical composition including SAC as an active ingredient and a pharmaceutically acceptable carrier for preventing or treating gastrointestinal disorders.

The present invention provides a food composition including SAC as an active ingredient for preventing or relieving gastrointestinal disorders.

The composition according to the present invention may further include a therapeutic agent for gastrointestinal disorders or an *anti-Helicobacter pylori* agent in addition to SAC.

The present invention provides a method of preventing or treating gastrointestinal disorders using a composition including SAC as an active ingredient and a pharmaceutically acceptable carrier.

Gastrointestinal disorders are also common in animals, and thus the present invention also provides a composition for animals.

The gastrointestinal disorders to which the composition according to the present embodiment may be applied include chronic gastritis, acute gastritis, gastric ulcer, gastric cancer, bleeding in the gastrointestinal tract, gastroesophageal reflux disease (GERD), duodentis, and duodenum ulcers.

In the composition according to the present embodiment, a pharmaceutically acceptable salt of SAC may be used as an active ingredient. The salt may be an acid addition salt or a quaternary ammonium salt. Examples of the acid addition salt include inorganic acid addition salts such as chloride, hydrobromide, hydroiodide, sulfate, and phosphate and organic acid addition salts such as oxalate, maleate, fumarate, lactate, malate, succinate, tartrate, benzoate, and methanesolfonate. Examples of the quaternary ammonium salt are a short-chain alkyl halogenide such as methyl iodide, methyl bromide, ethyl iodide, and ethyl bromide; a short-chain alkyl sulfonate such as methyl methanesulfonate and ethyl methanesulfonate; and a short-chain alkyl arylsulfonate such as methyl-*p*-toluenesulfonate.

The SAC or the pharmaceutically acceptable salt thereof may exist in a solvate or hydrate form, and thus a solvate or hydrate of the SAC or the pharmaceutically acceptable salt thereof may be used as an active ingredient for the therapeutic composition according to the present embodiment.

The SAC used herein may be prepared from a plant belonging to the Allium genus such as garlic, elephant garlic, onion, orscallion using a method disclosed in European Patent Publication No. EP 0429080A1, synthesis, fermentation, or any other known method.

The SAC, the pharmaceutically or sitologically acceptable salt of SAC, or a solvate or hydrate thereof, as an active ingredient, may be directly administered to patients. However, a composition including one or more of the active ingredients may be administered or a combined formulation prepared by mixing the active ingredients with an anti*-Helicobacter pylori* agent or a drug for treating gastrointestinal disorders may also be administered to patients.

The present invention provides a pharmaceutical composition formulated into a formulation for oral administration, a formulation for mucosal administration, an injection formulation, a formulation for inhalation, and a formulation for external application.

The formulation for oral administration may include hard and soft capsules, tablets, suspensions, powders, suspended-release formulations, enteric formulations, granules, oleosacchara, fine granules, pills, extracts, liquids, aromatic waters, emulsions, syrups, elixirs, fluid extracts, infusodecoctions, tinctures, medicated spirits, and infused oils.

The formulation for mucosal administration may be troches, buccal tablets, sublingual tablets, suppositories, and intranasal sprays, but is not limited thereto. The injection formulation may be subcutaneous injections, intramuscular injections, intravenous injections, and implant tablets.

The formulation for external application may be nasal drops, ophthalmic solutions, otic solutions, ophthalmic ointments, pastes, cataplasma, liniments, lotions, sprays, dusting powder, and liquids for external use.

The formulation according to the present embodiment may further include one or more inert carriers, in addition to one or more active ingredients, for example, excipients such as starch, lactose, carboxymethyl cellulose, and kaolin, binders such as water, gelatin, alcohol, glucose, gum Arabic, and gum Tragacanth, disintegrants such asstarch, dextrin, and sodium alginate, lubricants such as talc, stearic acid, magnesium stearate, and liquid paraffin, and other additives such as solubilizing agents.

A daily dose of SAC may vary according to various factors such as severity of disease, onset of the disease and a patient's age, condition, and complications. In general, a daily dose of SAC for an adult may be in the range of 1 mg to 10 g, preferably 100 mg to 4 g, and more preferably 200 to 2,000 mg. However, the daily dose may further increase for patients having severe symptoms or complications to improve therapeutic efficiency. The formulations may be administered in a single dose or divided into doses administered 2 or 3 times per day. For example, one or two unit dose formulations, each containing 200 to 500 mg of SAC may be orally administered once or twice per day, but the administration may be adjusted if required.

If the composition according to the present embodiment is a food composition, the amount of the active ingredients may be adjusted, if desired, for example, as disease-preventing or treating food or health supplements. In general, the amount of SAC in food or beverage may be in the range of 0.0001 to 90% by weight, preferably 0.1 to 50% by weight of the total food or beverage. Even though the amount of SAC in health supplements may be within the range described above for long-term use, it may be increased since the active ingredient is safe. Food including the food composition according to the present embodiment may be meat, sausage, bread, chocolate, candy, snack, pizza, instant noodle, gum, dairy products, soup, beverages, tea, drinks, alcohol, and vitamins.

Hereinafter, one or more embodiments will be described in detail with reference to the following examples. However, these examples are not intended to limit the purpose and scope of the invention.

### Experimental Examples

### Gastric Mucosal Protective Effect of SAC in Animals Having Gastric Lesions caused by Drugs

The gastric mucosal protective effect of SAC was evaluated in animals having gastric lesions induced by hydrochloric acid-ethanol, aspirin, or indomethacin.

### Test Material

SAC was purchased from TCI Chemical Co. (Tokyo, Japan). Sterile water for injection (Model No. 73H5F21, Dae Han Pharmaceutical Co. Ltd.) was used as a vehicle, and Stillen^{®} was used as a positive control material. 0.5% CMC-Na and sterile water for injection were used as excipients for Stillen^{®}. Hydrochloric acid was purchased from Samjung Chemical, Co., ethanol was purchased from Baker, Co., aspirin was purchased from Sigma, Co., and indomethacin was purchased from Sigma,Co.

### Test Animals

7 to 8-week old male specific pathogen free (SPF) HsdKoat:Sprague-Dawley^{®}™ SD^{®}™rats (weight of 7-week old males was in the range of 208.44 to 227.39 g, and weight of 8-week old males was in the range of 223.85 to 245.03 g, purchased from Koatech, Co. Ltd., Gyunggido, Korea) were quarantined and acclimated in the Animal Lab for 7 days. The rats were bred at a temperature of 23 3°C, at a relative humidity of 55 15%, with light for 12 hours (light turned on at 08:00 and light turned off at 20:00) while air was ventilated 10 to 20 times/hr in the Animal Experiment Lab of Gyeonggi Bio-Center. Other environments for breeding which might influence the test for the entire test period were not considered for use. The rats were given free access to a solid laboratory diet (Harlan Co. Ltd., USA. Teklad certified global 18% protein rodent diet, 2918C) that was supplied by Folas International. According to the analysis of certificate of diet composition, there were no ingredients or contaminants that could have had an adverse effect on the test. The rats were given free access to tap water that was sterilized using a UV sterilizer and a micro filter with water bottles.

### Test Group and Administration

The rats were classified into a vehicle control group G1 to which only a vehicle was administered, a experimental group G2 to which 100 mg/kg of SAC was administered, a experimental group G3 to which 200 mg/kg of SAC was administered, a experimental group G4 to which 400 mg/kg of SAC was administered, and a positive control group G5 to which 100 mg/kg of Stillen^{®} (55.6mg/kg as an active ingredient) as a positive control material were administered. Each group included 8 rats in a hydrochloric acid-ethanol-induced animal model (Experiment Example 7) and included 6 rats in an aspirin-induced animal model (Experimental Example 8) and in an indomethacin-induced animal model (Experimental Example 9).
Table 1

**[Table 1]**

| Group | **Gender** | **Total Number of animals** | **Number of animals** | **Administered Volume** (ml/kg) | **Dose** (mg/kg) |
|---|---|---|---|---|---|
| G1 | Male | 8(6) | 18∼(1∼6) | 10 | 0 |
| G2 | Male | 8(6) | 9∼16(7∼12) | 10 | 100 |
| G3 | Male | 8(6) | 17∼24(13∼18 ) | 10 | 200 |
| G4 | Male | 8(6) | 25∼32(19∼24 ) | 10 | 400 |
| G5 | Male | 8(6) | 33∼40(25∼30 ) | 10 | 55.6^{a)} |

| | | | | | |
|---|---|---|---|---|---|
| G1: Vehicle control group G2 to G4: Experimental groups to which SAC is administered G5: Positive control group to which a positive control material is administered a): Dose of active ingredient | | | | | |

The test substance was directly administered to the stomach using an injection tube equipped with a sonde for oral administration in a single dose once per day.

No animals died and no other changes were observed in any of the groups, and significant weight change with respect to the administration of the SAC was not observed at the administration and for the entire test period.

### Statistical Method

Comparisons of the vehicle control group with the experimental groups and the positive control material-administered experimental group were verified using one-way analysis of variance (One-way ANOVA). In this regard, significance and homoscedasticity were accepted, and thus a post-doc test was conducted using a Duncantest. Significance was accepted when p<0.05, and SPSS 10.1 was used.

### Experimental Example 7. Effect of SAC on Lengths of Gastric Lesions and Gastric Lesion Inhibiting Rate in Hydrochloric acid-ethanol-induced Gastric Lesion Animal Model

### Method

The test substances were administered. After one hour, 1.5 ml of 150 mM HCl in 60% ethanol was orally administered to each rat. The rats to which ethanol and SAC were administered were fasted without water in stainless steel breeding cages for 1 hour. After one hour from the hydrochloric acid-ethanol administration, the rats were sacrificed under anesthesia using ether and their stomachs including parts of the duodenum and esophagus were isolated. The insides of the stomachs were immediately washed with 13 ml of a 2% neutral buffered formalin, and the duodenum and esophagus parts were fixed with forceps. Then, 13 ml of the 2% neutral buffered formalin was added thereto and maintained for 5 minutes for fixation. The greater curvature of each stomach was incised, fixed to a dissection table, and unfolded to measure the lengths of gastric lesions using vernier calipers. Photographs of the unfolded stomachs were taken (FIG. 12) and the stomachs were fixed with a 10% neutral buffered formalin.

### Result

According to this gastric lesion model, ethanol directly stimulates gastric mucosa, induces edema in a muscle layer under a mucous membrane to cause a transient ischemic condition, and thus cell necrosis is induced due to oxidative damage, and hydrochloric acid directly stimulates the gastric mucosa and accelerates gastric motility to cause acute gastritis. By gross finding, the lesion was observed over the entire gastric mucosa and hemorrhage was observed in a long line. After one hour from the hydrochloric acid-ethanol administration, the rats of the vehicle control group had lesions over the entire gastric mucosa which was identified by atopsy (209.60±28.39 mm). The experimental group to which 200 mg/kg of SAC was administered (106.65±16.70 mm, p<0.01), the experimental group to which 400 mg/kg of SAC was administered (72.25±19.33 mm, p<0.01), and the positive control group to which Stillen^{®} was administered (102.51±11.35, p<0.01) exhibited significantly less lesions than the vehicle control group (refers to FIGS. 10 and 12).

Gastric lesion inhibiting rate (%)=(average length of the vehicle control group-length of gastric lesion of each animal)/average length of the vehicle control group X 100. The experimental group to which 200 mg/kg of SAC was administered (41.12±7.97%, p<0.01), the experimental group to which 400 mg/kg of SAC was administered (65.53±9.22%, p<0.01), and the positive control group to which Stillen^{®} was administered (51.09±5.41%, p<0.01) exhibited a significantly greater gastric lesion inhibiting rate (%) than the vehicle control group (refers to FIG. 11).

### Experimental Example 8. Effect of SAC on Area of Gastric Lesion and Gastric Lesion Inhibiting Rate in Aspirin-induced Gastric Lesion Animal Model

### Method

The rats were fasted for more than 24 hours in a normal environment, the test substance was administered, and 200 mg/kg of aspirin in 0.15 mol/L HCl was orally administered after 30 minutes. After three hours from the aspirin administration, the rats were sacrificed under ether anesthesia and their stomachs including parts of the duodenum and esophagus were isolated. The stomachs were fixed for 10 minutes by injecting 12 ml of 2% formalin. The greater curvature of each stomach was incised and unfolded, photographs of the gastric grandular region were taken, and then the areas of the lesions were measured using an image analyzer.

### Result

Table 2

**[Table 2]**

| **Group** | **Animal number(n=6)** | | | | | | **Ulcer Area** (mm²) | **SD** | **IR(%)** |
|---|---|---|---|---|---|---|---|---|---|
| | **1** | **2** | **3** | **4** | **5** | **6** | | | |
| G1 | 165.76 | 1555.7 7 | 216.14 | 231.8 8 | 285.7 2 | 236.6 7 | 215.3 | 48.35 | |
| G2 | 91.68 | 44.12 | 40.95 | 63.52 | 63.52 | 64.67 | 68.6 | 25.94 | -68.2 |
| G3 | 46.29 | 19.37 | 5.41 | 27.07 | 27.07 | 43.86 | 31.4 | 16.99 | -85.4 |
| G4 | 63.26 | 9.34 | 70.28 | 40.03 | 40.03 | 11.58 | 32.5 | 29.78 | -84.9 |
| G5 | 16.86 | 29.58 | 7.82 | 71.91 | 71.91 | 61.96 | 32.5 | 28.09 | -84.9 |

According to this gastric lesion model, aspirin that is a nonsteroidal anti-inflammatory drug inhibits the synthesis of prostaglandin that protects stomach walls, thereby causing gastric ulcers. The lesions were observed over the entire gastric mucosa and bleeding was observed in a net form. After aspirin administration, the rats of the vehicle control group had bleedings and lesions over the entire gastric mucosa which was identified by autopsy (215.3±48.35 mm²). The experimental group to which 100 mg/kg of SAC was administered (68.6±25.94 mm²), the experimental group to which 200 mg/kg of SAC was administered (31.4±16.99 mm²), the experimental group to which 400 mg/kg of SAC was administered (32.5±29.78 mm²), and the positive control group (32.5±28.09 mm²) exhibited significantly less lesions than the vehicle control group (refers to FIGS. 13 and 15).

Gastric lesion inhibiting rate (%)=(average area of the vehicle control group-area of gastric lesion of each animal)/average area of the vehicle control group X 100. The experimental group to which 100 mg/kg of SAC was administered (68.2%), the experimental group to which 200 mg/kg of SAC was administered (85.4%), the experimental group to which 400 mg/kg of SAC was administered (84.9%), and the positive control group (84.9%) exhibited a significantly greater gastric lesion inhibiting rate (%) than the vehicle control group (refer to FIG. 14).

### Experimental Example 9. Effect of SAC on Area of Gastric Lesion and Gastric Lesion Inhibiting Rate in Indomethacin-induced Gastric Lesion Animal Model

### Method

The rats were fasted for more than 24 hours in a normal environment, SAC was administered, and 25 mg/kg of indomethacin in distilled water was orally administered after 30 minutes. After six hours from the indomethacin administration, the rats were sacrificed under ether anesthesia and their stomachs including parts of the duodenum and esophagus were isolated. The stomachs were fixed for 10 minutes by injecting 12 ml of 2% formalin, the greater curvature of each stomach was incised and unfolded, photographs of the lesions were taken, and then the areas of the lesions were measured using an image analyzer.

### Result

Table 3

**[Table 3]**

| **Group** | **Animal number(n=6)** | | | | | | **Ulcer Area (mm²)** | **SD** | **IR(%)** |
|---|---|---|---|---|---|---|---|---|---|
| | **1** | **2** | **3** | **4** | **5** | **6** | | | |
| G1 | 6.4 | 18.0 | 6.7 | 4.2 | 0.9 | 9.6 | 7.6 | 5.85 | |
| G2 | 1.3 | 1.6 | 1.5 | 9.4 | - | 2.3 | 3.2 | 3.47 | -57.9 |
| G3 | 0.0 | 0.3 | 0.9 | 0.6 | 0.7 | 0.4 | 0.5 | 0.30 | -93.8 |
| G4 | 0.8 | 0.4 | 0.1 | 0.2 | 0.1 | 1.0 | 0.4 | 0.36 | -94.4 |
| G5 | 3.7 | 0.6 | 2.7 | 0.7 | 7.7 | 5.2 | 3.4 | 2.75 | -55.2 |

According to this gastric lesion model, indomethacin that is a nonsteroidal anti-inflammatory drug inhibits the synthesis of prostaglandin that protects stomach walls, thereby causing gastric lesions. Local lesions were observed in the gastric mucosa and bleeding was observed. After indomethacin administration, the rats of the vehicle control group had lesions having an area of 7.6±5.85 mm² which was identified by autopsy. In the experimental group to which 100 mg/kg of SAC was administered, the area of the lesions was 3.2±3.47mm² which was less than that of the vehicle control group by a half or more. The experimental group to which 200 mg/kg of SAC was administered (0.5±0.30 mm²), the experimental group to which 400 mg/kg of SAC was administered (0.4±0.36 mm²), and the positive control group (32.5±28.09 mm²) exhibited significantly less lesions than the vehicle control group. In particular, the experimental groups to which 200 mg/kg and 400 mg/kg of SAC were administered exhibited light gastric lesions and no bleedings (FIGS. 16 and 18).

The gastric lesion inhibiting rate (%) was calculated in the same manner as in Experimental Example 8. The experimental group to which 100 mg/kg of SAC was administered (57.9%), the experimental group to which 200 mg/kg of SAC was administered (93.8%), the experimental group to which 400 mg/kg of SAC was administered (94.4%), and the positive control group (55.2%) exhibited a significantly greater gastric lesion inhibiting rate (%) than the vehicle control group. Particularly, it was identified that the gastric lesion may be completely inhibited if 200 mg/kg or more of SAC is administered (refers to FIG. 17).

### Preparation Examples

Various formulations including SAC as an active ingredient were prepared as follows.

### Preparation Example 1. Manufacture of Tablet

| | |
|---|---|
| SAC | 200 mg |
| Lactose | 50 mg |
| Starch | 10 mg |
| Magnesium stearate | appropriate quantity |

The ingredients above were mixed and tableted using a known method to prepare a tablet.

### Preparation Example 2. Manufacture of Powder

| | |
|---|---|
| SAC | 250 mg |
| Lactose | 30 mg |
| Starch | 20 mg |
| Magnesium stearate | appropriate quantity |

The ingredients above were mixed and filled in a chartula coated with polyethylene, and the chartula was sealed to prepare powder.

### Preparation Example 3. Manufacture of Capsule

| | |
|---|---|
| SAC | 500 mg |
| Lactose | 30 mg |
| Starch | 28 mg |
| Magnesium stearate | appropriate quantity |

The ingredients above were mixed and filled in a gelatin hard capsule using a known method to prepare a capsule.

### Preparation Example 4. Manufacture of Suspension

| | |
|---|---|
| SAC | 50 mg |
| Isomerized sugar | 10 g |
| Sugar | 30 mg |
| Sodium carboxymethyl cellulose | 100 mg |
| Lemon flavor | appropriate quantity |
| Total volume including purified water | 100 ml |

The ingredients above were mixed to prepare a suspension using a known method and the suspension was filled in a 100 ml-brown bottle and sterilized.

### Preparation Example 5. Manufacture of Soft Capsule (amount in one soft capsule)

| | |
|---|---|
| SAC | 500 mg |
| Polyethylene glycol 400 | 400 mg |
| Concentrated glycerin | 55 mg |
| Purified water | 35 mg |

Polyethylene glycol and concentrated glycerin were mixed, and purified water was added thereto. Flavone was added thereto while the mixture was maintained at about 60°C, and the mixture was stirred at about 1,500 rpm with a stirrer. The mixture was cooled to room temperature while slowly stirring and bubbles were removed using a vacuum pump to prepare contents for a soft capsule. The coating of the soft capsule was prepared using gelatin and a plasticizer which are known in the art. 132 mg of gelatin, 52 mg of concentrated glycerin, 6 mg of 70% disobitol solution, an appropriate amount of ethyl vanillin as a flavoring agent, and carnauba wax as a coating base were used to prepare one soft capsule using a known method.

### Preparation Example 6. Manufacture of Injection

| | |
|---|---|
| SAC | 200 mg |
| Mannitol | 180 mg |
| Sterilized distilled water for injection | 2974 mg |
| Na₂HPO₄12H₂O | 26 mg |

An ample having the ingredients above was prepared using a known method.

### Preparation Example 7. Manufacture of Beverage

| | |
|---|---|
| SAC | 0.01 g |
| Citric acid | 8.5 g |
| White sugar | 10 g |
| Glucose | 2.5 g |
| DL-malic acid | 0.3 g |

| | |
|---|---|
| Purified water | appropriate quantity |

The ingredients above and an appropriate amount of purified water were mixed to a total volume of 100 mL and stirred to prepare a beverage using a known method.

## Claims

1. S-allyl-L-cysteine, a pharmaceutically acceptable salt of S-allyl-L-cysteine, or a solvate or hydrate thereof as an active ingredient for use in preventing or treating gastric mucosal lesions induced by a drug, wherein the drug is ethanol, aspirin or indomethacin.

2. S-allyl-L-cysteine, pharmaceutically acceptable salt of S-allyl-L-cysteine, or solvate or hydrate thereof for use according to claim 1, wherein the gastrointestinal disorder is chronic gastritis, acute gastritis, gastric ulcer, gastric cancer, bleeding in gastrointestinal tract, gastroesophageal reflux disease, duodentis or duodenum ulcer.

3. Pharmaceutical formulation comprising S-allyl-L-cysteine, pharmaceutically acceptable salt of S-allyl-L-cysteine, or solvate or hydrate thereof as an active ingredient for use in preventing or treating gastric mucosal lesions induced by a drug, wherein the drug is ethanol, aspirin or indomethacin according to claim 1, wherein said pharmaceutical formulation is selected from the group consisting of a formulation for oral administration, a formulation for mucosal administration, an injection formulation, a formulation for inhalation, and a formulation for external application.

4. Pharmaceutical formulation for use according to claim 3, wherein the formulation for oral administration is selected from the group consisting of hard and soft capsules, tablets, suspensions, powder, suspended-release formulations, enteric formulations, granules, oleosacchara, fine granules, pills, extracts, liquids, aromatic waters, emulsions, syrups, elixirs, fluid extracts, infusodecoctions, tinctures, medicated spirits, and infused oils.

## Patentansprüche

1. S-Allyl-L-Cystein, ein pharmazeutisch akzeptables Salz von S-Allyl-L-Cystein, oder ein Solvat oder Hydrat desselben als ein Wirkstoff zur Verwendung beim Verhindern oder Behandeln von durch ein Medikament verursachten Magenschleimhautläsionen, wobei das Medikament Ethanol, Aspirin oder Indomethacin ist.

2. S-Allyl-L-Cystein, ein pharmazeutisch akzeptables Salz von S-Allyl-L-Cystein oder ein Solvat oder Hydrat desselben zur Verwendung nach Anspruch 1, wobei die gastrointestinale Störung eine chronische Gastritis, eine akute Gastritis, ein Magengeschwür, ein Magenkrebs, eine Blutung im Gastrointestinaltrakt, eine gastroösophageale Refluxkrankheit, Duodenitis oder ein Zwölffingerdarmgeschwür ist.

3. Pharmazeutische Zusammensetzung umfassend S-Allyl-L-Cystein, ein pharmazeutisch akzeptables Salz von S-Allyl-L-Cystein oder ein Solvat oder Hydrat desselben als Wirkstoff zur Verwendung beim Verhindern oder Behandeln von durch ein Medikament verursachten Magenschleimhautläsionen, wobei das Medikament Ethanol, Aspirin oder Indomethacin ist, nach Anspruch 1, wobei die pharmazeutische Zusammensetzung ausgewählt ist aus der Gruppe bestehend aus einer Zusammensetzung zur oralen Verabreichung, einer Zusammensetzung zur mukosalen Verabreichung, einer Injektionsverabreichung, einer Inhalationsverabreichung und einer Verabreichung für eine externe Anwendung.

4. Pharmazeutische Zusammensetzung zur Verwendung nach Anspruch 3, wobei die Zusammensetzung zur oralen Verabreichung ausgewählt ist aus der Gruppe bestehend aus harten und weichen Kapseln, Tabletten, Suspensionen, Pulvern, Zusammensetzungen mit verzögerter Freisetzung, enteralen Zusammensetzungen, Granulaten, Oleosacchara, feinen Granulaten, Pillen, Extrakten, Flüssigkeiten, aromatischen Wässern, Emulsionen, Sirupen, Elixieren, Fluidextrakten, Infusodecoctionen, Tinkturen, Medikament-Spirituosen und versetzte Ölen.

## Revendications

1. S-allyl-L-cystéine, sel pharmaceutiquement acceptable de S-allyl-L-cystéine, ou solvate ou hydrate de celle-ci en tant qu'ingrédient actif pour utilisation dans la prévention ou le traitement de lésions muqueuses gastriques induites par une drogue ou un médicament, la drogue ou le médicament étant l'éthanol, l'aspirine ou l'indométacine.

2. S-allyl-L-cystéine, sel pharmaceutiquement acceptable de S-allyl-L-cystéine, ou solvate ou hydrate de celle-ci pour utilisation selon la revendication 1, dans laquelle le trouble gastro-intestinal est une gastrite chronique, une gastrite aiguë, un ulcère gastrique, un cancer gastrique, un saignement des voies gastro-intestinales, une maladie du reflux gastro-oesophagien, une duodénite ou un ulcère du duodénum.

3. Formulation pharmaceutique comprenant de la S-allyl-L-cystéine, un sel pharmaceutiquement acceptable de S-allyl-L-cystéine, un solvate ou hydrate de celle-ci en tant qu'ingrédient actif pour utilisation dans la prévention ou le traitement de lésions muqueuses gastriques induites par une drogue ou un médicament, la drogue ou le médicament étant l'éthanol, l'aspirine ou l'indométacine selon la revendication 1, dans laquelle ladite formulation pharmaceutique est choisie dans le groupe constitué d'une formulation pour administration orale, d'une formulation pour administration par la muqueuse, d'une formulation pour injection, d'une formulation pour inhalation et d'une formulation pour application externe.

4. Formulation pharmaceutique pour utilisation selon la revendication 3, dans laquelle la formulation pour administration orale est choisie dans le groupe constitué par les capsules et gélules dures et molles, les comprimés, les suspensions, une poudre, les formulations à libération retardée, les formulations entériques, les granules, les oléosaccharures, les granules fins, les pilules, les extraits, les liquides, les eaux aromatiques, les émulsions, les sirops, les élixirs, les extraits de fluide, les infuso-décoctions, les teintures, les eaux de vie médicamenteuses et les huiles infusées.
